(19) Europäisches Patentamt
European Patent Office
Office européen des brevets

(11) **EP 1 316 310 B1**

(12) **EUROPEAN PATENT SPECIFICATION**

(45) Date of publication and mention of the grant of the patent:
**09.07.2008  Bulletin 2008/28**

(51) Int Cl.:
*A61K 31/417* (2006.01)     *A61K 9/08* (2006.01)
*A61P 27/02* (2006.01)

(21) Application number: **02026095.6**

(22) Date of filing: **22.11.2002**

(54) **Ophthalmic composition comprising a histidine derivative for healing asthenopia**

Ophtalmische Zubereitung enthaltend ein Histidin Derivat zur Behandlung des gereizten Auges

Composition Ophtalmique contenant un dérivé de la histidine pour le traitement de l'irritation occulaire

(84) Designated Contracting States:
**AT BE BG CH CY CZ DE DK EE ES FI FR GB GR IE IT LI LU MC NL PT SE SK TR**

(30) Priority: **30.11.2001  JP 2001366647**

(43) Date of publication of application:
**04.06.2003  Bulletin 2003/23**

(73) Proprietor: **Menicon Co., Ltd.**
**Nagoya-shi**
**Aichi (JP)**

(72) Inventors:
• **Tsuzuki, Akira**
  **Nagoya-shi,**
  **Aichi (JP)**
• **Tanikawa, Sadayasu**
  **Kasugai-shi,**
  **Aichi (JP)**

(74) Representative: **Wächtershäuser, Günter et al**
**Wächtershäuser & Hartz**
**Weinstrasse 8**
**80333 München (DE)**

(56) References cited:
WO-A-02/04005         FR-A- 2 701 947
US-A- 5 792 784

• **DATABASE WPI Section Ch, Week 199417 Derwent Publications Ltd., London, GB; Class B04, AN 1994-141007 XP002230427 & JP 06 087887 A (UPJOHN CO), 29 March 1994 (1994-03-29)**
• **BABIZHAYEV M A ET AL: "THE NATURAL HISTIDINE-CONTAINING DIPEPTIDE NALPHA-ACETYLCARNOSINE AS AN ANTIOXIDANT FOR OPHTHALMIC USE" BIOCHEMISTRY, vol. 65, no. 5, May 2000 (2000-05), pages 588-598, XP008004143**
• **BABIZHAYEV M A ET AL: "NALPHA-ACETYLCARNOSINE IS A PRODRUG OF L-CARNOSINE IN OPHTHALMIC APPLICATION AS ANTIOXIDANT" CLINICA CHIMICA ACTA, AMSTERDAM, NL, vol. 254, no. 1, 15 October 1996 (1996-10-15), pages 1-21, XP001074750 ISSN: 0009-8981**
• **DATABASE MEDLINE [Online] November 1997 (1997-11) MAICHUK IU F ET AL: "[Development of carnosine eyedrops and assessing their efficacy in corneal diseases]" Database accession no. NLM9483997 XP002230426 & VESTNIK OFTALMOLOGII. RUSSIA 1997 NOV-DEC, vol. 113, no. 6, November 1997 (1997-11), pages 27-31, ISSN: 0042-465X**

Remarks:
The file contains technical information submitted after the application was filed and not included in this specification

**Description**

The present invention relates to an ophthalmic composition, particularly to an ophthalmic composition for healing asthenopia, which is sufficiently provided with safety to eyes and which is capable of effectively healing asthenopia caused by wearing contact lenses or by VDT (visual display terminal) operation, especially an eye drop or an agent for contact lenses such as a cleaning fluid for contact lenses.

Heretofore, various types of eye drops containing various components, have been proposed for the purpose of releasing or healing asthenopia or eye strain accumulated by an operation in which eyes are overused, such as VDT operation, or reading, watching or observing operation.

Biochemistry (Moscow) 65 (2000) 588-598 and Clinica Chimica Acta 254 (1996) 1-21 disclose the use of acetyl-carnosine for treating eye diseases associated with oxidative stress. Vestnik oftalmologii. 113 (1997) 27-31 discloses the use of carnosine for treating corneal diseases.

For example, as an eye drop to improve the effect for healing asthenopia by combining taurine with a prescribed specific component, JP-A-9-40549 proposes an eye drop for healing asthenopia, which comprises taurine and menthol, and JP-A-9-143064 discloses an eye drop for healing asthenopia, which comprises taurine, menthol, neostigmine methylsulfate and VE acetate.

However, with such eye drops, the effect of healing asthenopia can hardly be regarded as sufficient, and it is still desired to develop an eye drop which presents a better effect for healing asthenopia.

On the other hand, as an eye drop intended to heal asthenopia by having other component different from taurine incorporated, JP-A-2000-247885 proposes an eye drop for healing asthenopia, which contains caffeine as an active ingredient, and various other eye drops are also proposed which contain a vasoconstrictor which acts on sclerotic vein in the eyeball to release inflammation of the eye and which is effective for healing asthenopia.

However, caffeine is addictive and is a substance designated as a deleterious substance. On the other hand, the vasoconstrictor is said to have a danger of a so-called rebound symptom such that if its use is discontinued, inflammation of the eye returns, or asthenopia tends to be amplified. Accordingly, it is not desirable to use an eye drop or the like containing such a component, when its influence to the human body is taken into consideration.

Under these circumstances, the present invention has been made, and it is an object of the present invention to provide an ophthalmic composition for healing asthenopia, which is sufficiently provided with safety to eyes and which is capable of effectively healing asthenopia, particularly specifically an eye drop or an agent for contact lenses such as a cleaning fluid for contact lenses.

To accomplish such an object, the present invention provides an ophthalmic composition characterized in that at least one histidine derivative is incorporated in an aqueous medium, whereby the histidine derivative is anserine incorporated in a proportion of from 0.001 to 5 w/w%.

Thus, the ophthalmic composition of the present invention is one having a histidine derivative incorporated which is safe to living organism and is capable of providing excellent effect for healing asthenopia, and it has a feature that it is capable of effectively releasing or reducing asthenopia or eye strain caused by e.g. wearing contact lenses or VDT operation in which eyes are overused.

Further, in one of preferred embodiments of the ophthalmic composition according to the present invention, anserine and carnosine is advantageously used as the above histidine derivative, whereby a safer and more effective effect for healing asthenopia can be obtained.

Further, according to another preferred embodiment of the present invention, the above anserine is incorporated in a proportion of from 0.001 to 5 w/w%, while the above carnosine is incorporated in a proportion of from 0.0001 to 3 w/w%. By adopting such a content, it is possible to obtain an excellent effect for healing asthenopia, while advantageously securing the safety to eyes and without changing the specification of the contact lens when it is applied while the contact lens is put on.

Further, in such an ophthalmic composition of the present invention, preferably, a thickener and/or a surfactant is further incorporated in addition to the histidine derivative, whereby the histidine derivative can be retained for a longer period of time in the conjunctiva sac or on the cornea of the eyeball, and thus, a superior effect for healing asthenopia will be provided.

Further, in the ophthalmic composition according to the present invention, at least one member selected from the group consisting of antiseptics, buffer agents, chelating agents, isotonic agents, refrigerants, antiinflammatory, vitamins and amino acids, may further be incorporated as the case requires, in addition to the above-described respective components, whereby further functions corresponding to such components, such as functions to preserve contact lenses, will be advantageously added.

Still further, according to one of preferred embodiments of the present invention, the above-mentioned ophthalmic composition is one which is sufficiently provided with safety to eyes and thus is preferably employed as an eye drop which can be directly applied to the eyes of e.g. a VDT operator suffering from asthenopia thereby to effectively healing such asthenopia. Further, when it is applied while a contact lens is put on, it not only heals asthenopia but also effectively improves the comfortableness of wearing the contact lens.

In addition, according to another preferred embodiment of the present invention, the above-mentioned ophthalmic composition is not one which changes the specification of contact lenses, and it can be preferably employed as an agent for contact lenses such as a cleaning fluid for contact lenses, a rinsing fluid for contact lenses or a preservation fluid for contact lenses.

Such an ophthalmic composition of the present invention contains at least one histidine derivative as an essential component and has a significant feature in that it is constructed so that the desired effective effects can be obtained by incorporating the histidine derivative in an aqueous medium.

Specifically, such a histidine derivative to be used in the present invention, is safe to a living organism and is a highly effective anti-asthenopic substance. Accordingly, by administering the ophthalmic composition of the present invention having at least one of such histidine derivatives incorporated, to an eye having a lactic acid value increased by a stress of e.g. wearing a contact lens or VDT operation for a long period of time, and showing a symptom of asthenopia, by a method such as application of eye drop, it is possible to effectively release or reduce asthenopia without presenting any adverse effect to the living organism.

Here, as such histidine derivatives, any histidine derivatives may be employed so long as they have anti-asthenopic effects to living organisms. However, among them, anserine and/or carnosine may be used particularly advantageously which has excellent anti-asthenopic effects or anti-oxidation effects, and by using such specific histidine derivatives, better effects for healing asthenopia can be obtained.

Further, the amount of such a histidine derivative to be used, is optionally determined in consideration of overall aspects including the desired degree for healing asthenopia, production cost, etc. In a case where the above-mentioned anserine and/or carnosine is used, if the proportion is too small, no adequate effect for improving asthenopia can be obtained, and inversely, if it is too large, irritation to the eye is likely to be induced, or when it is applied while a contact lens is put on, it may change the specification of the contact lens such as the lens diameter or the base curve. Accordingly, it is common to employ anserine at a concentration of from 0.001 to 5 w/w%, preferably from 0.01 to 3 w/w%, or carnosine at a concentration of from 0.0001 to 3 w/w%, preferably from 0.001 to 1 w/w%.

At the time of incorporating the histidine derivative, an isolated histidine derivative may be added and incorporated to a proper aqueous medium which will be described hereinafter, or a mixture containing two or more histidine derivatives may be added and incorporated. And, as such a mixture containing two or more histidine derivatives, Marine Active (tradename, produced by Yaizu Suisan Kagaku Industry Co., Ltd.) containing anserine and carnosine, extracted from a fishery product such as tuna or bonito, may advantageously be used.

In the ophthalmic composition of the present invention, a thickener and/or a surfactant may further be incorporated in addition to the above-mentioned at least one histidine derivative, whereby the effect for healing asthenopia will more effectively be provided.

Namely, if the ophthalmic composition having a thickener and/or a surfactant incorporated together with the histidine derivative, is administered to an eye by a method such as application of eye drop, the histidine derivative as a component to heal asthenopia, will be retained for a longer period of time in the conjunctiva sac or on the cornea, by the presence of the thickener or the surfactant. Further, in a case where it is applied while a contact lens is put on, the histidine derivative not only will be retained in the conjunctiva sac or on the cornea but also will deposit on the contact lens surface or will be adsorbed mildly in the interior of the contact lens, whereby the histidine derivative as a component to heal asthenopic, will be gradually released on the cornea, and the effect for healing asthenopia by the histidine derivative will be continuously provided.

Here, such thickener and surfactant are preferably ones which have high safety to living organisms and are sufficiently allowable ophthalmologically and which are non-influential to the shape or physical properties of contact lenses, and they are preferably employed in an amount within a range to satisfy such requirements.

Specifically, as the thickener, it is possible to suitably employ, for example, various gums such as mucopolysaccharides or heteropolysaccharides; a synthetic organic polymer compound such as polyvinyl alcohol, poly-N-vinylpyrrolidone, polyethylene glycol, polypropylene glycol or polyacrylamide; a cellulose derivative such as hydroxyethylcellulose, hydroxypropylmethylcellulose, carboxymethylcellulose or methylcellulose; or a starch derivative.

Further, as the surfactant, it is possible to advantageously employ, for example, a polyoxyethylene/polyoxypropylene block copolymer or its derivative, a polyethylene glycol derivative such as a polyoxyethylene alkylphenyl ether/ formaldehyde condensate like Tyloxapol, a sorbitan fatty acid ester such as sorbitan sesquioleate or sorbitan monooleate like Polysorbate 80, a polyoxyethylene sorbitan fatty acid ester such as polyoxyethylene sorbitan monooleate, a glycerol fatty acid ester such as glyceryl monostearate, a polyethylene glycol fatty acid ester such as polyethylene glycol monostearate, a polyoxyethylene alkyl ether such as polyoxyethylene lauryl ether, or a polyoxyethylene castor oil. Among them, it is particularly preferred to employ Pluronic, Pluronic R, Tetronic or Tetronic R (manufactured by BASF, Germany) as a polyoxyethylene/polyoxypropylene block copolymer commercially available as a nonionic surfactant, specifically, Poloxamer 124, Poloxamer 188, Poloxamer 237, Poloxamer 338, Poloxamer 407, Tetronic 904, Tetronic 908, Tetronic 1103, Tetronic 1107, or Polysorbate 80 as sorbitan monooleate.

Further, to the ophthalmic composition of the present invention, one or more of various additive components which are commonly used in eye drops or in agents for contact lenses may be incorporated within a commonly employed range, as the case requires, in addition to the above-described components. Such additive components are preferably those which have high safety to living organisms and are sufficiently allowable ophthalmologically and which are not influential over the shape or physical properties of contact lenses, and they are preferably employed in an amount within a range to satisfy such requirements, whereby it is possible to advantageously impart various functions corresponding to the respective additive components to the ophthalmic composition without adversely affecting the effects of the present invention.

Further, to the ophthalmic composition of the present invention, an antiseptic having antiseptic effects, may be incorporated to advantageously provide sterilizing effects for eyes or contact lenses, or antiseptic and preservative effects for the ophthalmic composition. As such an antiseptic, proper ones may be selected among various known antiseptics and used alone or in combination as a mixture of a plurality of them.

Such an antiseptic may, for example, be sorbic acid, potassium sorbate, benzoic acid or its salt, ethyl p-oxybenzoate, butyl p-oxybenzoate, propyl p-oxybenzoate, methyl p-oxybenzoate or chlorobutanol.

Further, with the ophthalmic composition according to the present invention, if the pH value or the osmotic pressure is too high or too low, it is likely to present irritation to eyes or to bring about a trouble of eyes. Accordingly, the pH value of such an ophthalmic composition is usually preferably adjusted to a level of from 5.3 to 8.5 by an addition of a suitable pH adjusting agent. Likewise, the osmotic pressure is preferably adjusted to a level of from 200 to 400 mOsm/kg by an addition of an isotonic agent. As such a pH adjusting agent to be used for adjusting the pH, sodium hydroxide or hydrochloric acid may, for example, be used. Whereas, as an isotonic agent to be used for adjusting the osmotic pressure, at least one compound selected from the group consisting of sodium chloride, potassium chloride, saccharides, sugar alcohol, and polyhydric alcohols or their ethers or esters, may usually be employed.

Further, it is common to incorporate at least one buffer agent in order to maintain the pH of such an ophthalmic composition within the above-mentioned range and within a range safe to eyes. Such a buffer agent may be suitably selected and employed among various types of conventional buffer agents. Specifically, an acid such as phosphoric acid, boric acid, a carboxylic acid or an oxycarboxylic acid, or its salt (such as a sodium salt), or Good-Buffer, tris (hydroxymethyl)aminomethane(TRIS), bis(2-hydroxyethyl)iminotris(hydroxymethyl)methane(Bis-Tris), or sodium hydrogencarbonate, may, for example, be mentioned in view of the safety to eyes and with a view to minimizing the influence to contact lenses.

Furthermore, with contact lenses, particularly soft contact lenses, there is a possibility that as a stain from lacrimal fluid, calcium or the like is likely to deposit or be adsorbed. To prevent such a trouble, a chelating agent may advantageously be added to the ophthalmic composition. As such a chelating agent, ethylenediamine tetraacetic acid (EDTA) or its salt, such as disodium ethylenediamine tetraacetate (EDTA·2Na) or trisodium ethylenediamine tetraacetate (EDTA·3Na), may, for example, be employed.

In addition, a refrigerant such as menthol, borneol, camphor, geraniol, eucalyptus oil, bergamot oil, fennel oil, peppermint oil, rose oil or cool mint, may be incorporated for the purpose of presenting refreshing sensation at the time of application of eye drop or releasing irritating or itching sensation when a contact lens is put on. Further, an antiinflammatory such as glycyrrhizinic acid or its salt, $\varepsilon$-aminocaproic acid, allantoin or sodium azulenesulfonate, may also be incorporated in order to control intraocular inflammation caused by stress or by wearing a contact lens.

Further, depending upon the intended application of the ophthalmic composition, vitamins such as vitamins A (inclusive of retinol palmitate and $\beta$-carotene), vitamins $B_2$, $B_6$ and $B_{12}$ and vitamins E such as d-$\alpha$-tocopherol acetate, or pantenol, asparaginic acid and its salt, and amino acids such as aminoethylsulfonic acid, arginine, alanine, lysine and glutamic acid, may also be optionally added.

Such an ophthalmic composition according to the present invention is prepared by adding and incorporating the above-described components in the respective proper amounts in a suitable aqueous medium in the same manner as heretofore. As the aqueous medium to be used, in addition to water itself such as city water, purified water or distilled water, a solution composed mainly of water may be employed such as saline water, an aqueous sodium chloride solution, an eye drop or a conventional agent for contact lenses.

When the ophthalmic composition of the present invention thus obtained, is applied to an eye suffering from asthenopia, the ophthalmic composition may be used as an eye drop and applied in a suitable amount in the same manner as heretofore, irrespective of whether or not a contact lens is put on, or especially for a person wearing a contact lens, a method may be employed in which the contact lens is washed or rinsed with such an ophthalmic composition, and then such a contact lens is put on, or a contact lens is washed with another washing fluid for contact lenses, and it is preserved in such an ophthalmic composition for a predetermined period of time, and then the contact lens is put on.

By applying the phthalmic composition of the present invention to a VDT operator or a contact lens user suffering from asthenopia as described above, the histidine derivative as a component to heal asthenopia, will effectively be supplied into the eyeball, whereby asthenopia will be effectively healed, or for the contact lens user, not only such asthenopia will be healed, but also the comfortableness in wearing a contact lens will be improved.

Besides, such a histidine derivative has high safety to living organisms. Accordingly, there is a merit in that a problem such as a trouble to an eye will not be brought about even if the ophthalmic composition is used continuously over a long period of time.

Further, the ophthalmic composition of the present invention will not change the specification of contact lenses, and will not hinder the effects of a disinfectant, a cleaning agent, etc. which are commonly contained in an agent for contact lenses. Accordingly, such an ophthalmic composition can advantageously be used as an agent for contact lenses, excellent in the effect for healing asthenopia.

Further, it is needless to say that such an agent for contact lenses can be used not only for a single purpose such as cleaning, rinsing or preserving contact lenses but also as a multipurpose agent for contact lenses, for example, for the purpose of rinsing and preserving cleaned contact lenses (multipurpose solution).

Further, the type of contact lenses to be treated by the ophthalmic composition of the present invention is not particularly limited, and for example, soft contact lenses and hard contact lenses which may be classified in all of low water content or high water content contact lenses, may be thereby treated. Further, the material of contact lenses or the like, may not be any problem in the application of the present invention.

Now, the present invention will be described in further detail with reference to some Examples and Test Examples of the present invention.

TEST EXAMPLE 1

Various liquid samples (Sample Nos. 1 to 54) having a pH of 6.8 were respectively prepared by adding prescribed components in the respective proportions as identified in the following Tables 1 to 8 to sterilized purified water. For the preparation of such liquid samples, as the histidine derivative being an essential component for healing asthenopia in the present invention, isolated L-carnosine, or "Marine Active" tradename, manufactured by Yaizu Suisan Kagaku Industry Co., Ltd., containing anserine and carnosine (anserine content: 6.3 w/w%, carnosine content: 0.3 w/w%) was used. Further, as the surfactant, Poloxamer 407 (Px407, manufactured by BASF, molecular weight: 8,000-12,000), Tetronic 1107 (manufactured by BASF, average molecular weight: 15,000) or polyoxyethylene (20) sorbitan monooleate (Polysorbate, manufactured by Nikko Chemicals K.K., TO-10M) was used, and as the thickener, hydroxypropylmethylcellulose (HPMC), hydroxyethylcelullose, sodium hyaluronate or sodium chondroitin sulfate, was used. Further, disodium ethylenediamine tetraacetate (EDTA·2Na) as a chelating agent, boric acid or borate as a buffer agent, potassium solvate or polyhexamethylene biguanide (PHMB) as a disinfectant (antiseptic), sodium chloride, potassium chloride or propylene glycol as an isotonic agent, and 1-menthol or dl-camphor as a refrigerant, were, respectively, employed as the case required. And, with respect to the obtained respective liquid samples, the after-described test on the effect for healing asthenopia and test on the compatibility with lenses, were carried out.

Test on the effect for healing asthenopia

The following sensory test was carried out to a total of 30 volunteers comprising 10 rigid gas permeable hard contact lens users, 10 soft contact lens users and 10 VDT operators not wearing contact lenses. Specifically, to each volunteer, from 1 to 3 drops of a liquid sample per each time was applied at a frequency of from 3 to 6 times per day during the test period of one month, and the volunteer himself or herself evaluated the effect for healing asthenopia by each liquid sample during the test period, whereupon the evaluations relating to the respective liquid samples were collected, and the results are also shown in the following Tables 1 to 8.

Here, in each of the following Tables, "◎◎" indicates a case where the proportion of volunteers who answered that asthenopia due to wearing of a contact lens or due to VDT operation was remarkably reduced, was at least 90%; "◎" indicates a case where the proportion of volunteers who answered that asthenopia was remarkably reduced, was at least 70% and less than 90%; "○" indicates a case where the proportion of volunteers who answered that asthenopia was remarkably reduced, was at least 50% and less than 70%; and "Δ" indicates a case where the proportion of volunteers who answered that asthenopia was remarkably reduced, was less than 50%.

Test on the compatibility with lenses

The following test was carried out to examine the compatibility with contact lenses of the ophthalmic composition of the present invention. Namely, firstly, a plurality of soft contact lenses (Menicon Soft 72, manufactured by Menicon Co., Ltd., lens diameter: 13.5 mm) were prepared, and they were immersed in physiological saline (stipulated by ISO 10344) maintained at a temperature of 25˚C. Then, in the immersed state, the lens diameters of the respective contact lenses were measured by means of a profile projector (a universal projector, manufactured by Nikon Corporation), and the obtained measured values were recorded as the initial values of the lens diameters, respectively.

Then, the contact lenses having the initial values of lens diameters thus measured, were immersed in the liquid samples prepared as described above, under a condition of a temperature of 25°C for 3 days. Then, in such an immersed state, the lens diameters were measured by the same profile projector as mentioned above. With respect to each liquid sample, three contact lenses were used for such an operation.

And, the difference (d) between the measured value (the value after immersion) of the lens diameter thus obtained and the initial value of the lens diameter previously obtained, was calculated with respect to each contact lens in accordance with the formula: d=the value after immersion-the initial value, and the average value of such calculated values was calculated for every liquid sample. Here, it is needless to say that the smaller the value of the change in the lens diameter, the better the compatibility with contact lenses. Further, the value of the change in the lens diameter is desired to be within ±0.2 mm. Accordingly, the liquid sample whereby the value of the change in the lens diameter was within ±0.2 mm, was evaluated to be "acceptable", while one whereby the same value is larger than ±0.2 mm was evaluated to be "not acceptable". Such evaluations were also shown in Tables 1 to 8.

Table 1

| Incorporated components | Sample No. | | | | | |
|---|---|---|---|---|---|---|
| | 1 | 2 | 3 | 4 | 5 | 6 |
| L-carnosine | - | - | - | - | - | 1 |
| Marine Active | 1 | 1 | 1 | 1 | 1 | - |
| Px407 | - | - | - | - | - | 0.5 |
| Hydroxypropyl-methylcellulose | - | 0.15 | - | - | - | 0.15 |
| Hydroxyethyl-cellulose | - | - | 0.15 | - | - | - |
| Sodium hyaluronate | - | - | - | 0.15 | - | - |
| Sodium chondroitin sulfate | - | - | - | - | 0.5 | - |
| EDTA·2Na | 0.05 | 0.05 | 0.05 | 0.05 | 0.05 | 0.05 |
| Boric acid | 0.5 | 0.5 | 0.5 | 0.5 | 0.5 | 0.5 |
| Borax | 0.05 | 0.05 | 0.05 | 0.05 | 0.05 | 0.05 |
| Potassium sorbate | 0.15 | 0.1 | 0.1 | 0.1 | 0.1 | 0.1 |
| NaCl | 0.275 | 0.275 | 0.275 | 0.275 | 0.275 | 0.275 |
| KCl | 0.055 | 0.055 | 0.055 | 0.055 | 0.055 | 0.055 |
| Effect for healing asthenopia | ○ | ◎ | ◎ | ◎ | ◎ | ◎◎ |
| Test on compatibility with lenses | AC | AC | AC | AC | AC | AC |
| AC: Acceptable * Incorporated proportion: w/w% | | | | | | |

Table 2

| Incorporated components | Sample No. | | | | | | | |
|---|---|---|---|---|---|---|---|---|
| | 7 | 8 | 9 | 10 | 11 | 12 | 13 | 14 |
| Marine Active | 1 | 1 | 1 | 1 | 1 | 1 | 1 | 1 |
| Px407 | 0.5 | - | - | - | 0.5 | - | 0.5 | - |
| Tetronic 1107 | - | 0.5 | - | - | - | 0.5 | - | 0.5 |
| Polysorbate | - | - | 0.25 | - | - | - | - | - |
| Hydroxypropyl-methylcellulose | 0.15 | 0.15 | 0.15 | 0.15 | - | - | - | - |
| Hydroxyethyl-cellulose | - | - | - | - | 0.15 | 0.15 | - | - |
| Sodium hyaluronate | - | - | - | - | - | - | 0.15 | 0.15 |
| EDTA·2Na | 0.05 | 0.05 | 0.05 | 0.05 | 0.05 | 0.05 | 0.05 | 0.05 |

(continued)

| Incorporated components | Sample No. | | | | | | | |
|---|---|---|---|---|---|---|---|---|
| | 7 | 8 | 9 | 10 | 11 | 12 | 13 | 14 |
| Boric acid | 0.5 | 0.5 | 0.5 | 0.5 | 0.5 | 0.5 | 0.5 | 0.5 |
| Borax | 0.05 | 0.05 | 0.05 | 0.05 | 0.05 | 0.05 | 0.05 | 0.05 |
| Potassium sorbate | 0.1 | 0.1 | 0.1 | 0.1 | 0.1 | 0.1 | 0.1 | 0.1 |
| NaCl | 0.275 | 0.275 | 0.275 | 0.085 | 0.275 | 0.275 | 0.275 | 0.275 |
| KCl | 0.055 | 0.055 | 0.055 | 0.017 | 0.055 | 0.055 | 0.055 | 0.055 |
| Propylene glycol | - | - | - | 0.5 | - | - | - | - |
| Effect for healing asthenopia | ◎◎ | ◎◎ | ◎◎ | ◎◎ | ◎◎ | ◎◎ | ◎◎ | ◎◎ |
| Test on compatibility with lenses | AC | AC | AC | AC | AC | AC | AC | AC |
| AC: Acceptable<br>* Incorporated proportion: w/w% | | | | | | | | |

Table 3

| Incorporated components | Sample No. | | | | | |
|---|---|---|---|---|---|---|
| | 15 | 16 | 17 | 18 | 19 | 20 |
| Marine Active | 1 | 1 | 1 | 1 | 1 | 1 |
| Px407 | 0.5 | - | - | - | 0.5 | - |
| Tetronic 1107 | - | 0.5 | - | - | - | 0.5 |
| Polysorbate | - | - | 0.25 | - | - | - |
| Hydroxypropyl-methylcellulose | 0.15 | 0.15 | 0.15 | 0.15 | - | - |
| Hydroxyethyl-cellulose | - | - | - | - | 0.15 | 0.15 |
| EDTA·2Na | 0.05 | 0.05 | 0.05 | 0.05 | 0.05 | 0.05 |
| Boric acid | 0.5 | 0.5 | 0.5 | 0.5 | 0.5 | 0.5 |
| Borax | 0.05 | 0.05 | 0.05 | 0.05 | 0.05 | 0.05 |
| PHMB | 0.0001 | 0.0001 | 0.0001 | 0.0001 | 0.0001 | 0.0001 |
| NaCl | 0.275 | 0.275 | 0.275 | 0.085 | 0.275 | 0.275 |
| KCl | 0.055 | 0.055 | 0.055 | 0.017 | 0.055 | 0.055 |
| Propylene glycol | - | - | - | 0.5 | - | - |
| Effect for healing asthenopia | ◎◎ | ◎◎ | ◎◎ | ◎◎ | ◎◎ | ◎◎ |
| Test on compatibility with lenses | AC | AC | AC | AC | AC | AC |
| AC: Acceptable<br>* Incorporated proportion: w/w% | | | | | | |

Table 4

| Incorporated components | Sample No. | | | | | | | |
|---|---|---|---|---|---|---|---|---|
| | 21 | 22 | 23 | 24 | 25 | 26 | 27 | 28 |
| Marine Active | 1 | 1 | 1 | 1 | 1 | 1 | 1 | 1 |
| Px407 | 0.5 | - | - | - | 0.5 | - | - | - |

(continued)

| Incorporated components | Sample No. | | | | | | | |
|---|---|---|---|---|---|---|---|---|
| | 21 | 22 | 23 | 24 | 25 | 26 | 27 | 28 |
| Tetronic 1107 | - | 0.5 | - | - | - | 0.5 | - | - |
| Polysorbate | - | - | 0.25 | - | - | - | 0.25 | - |
| Hydroxypropyl-methylcellulose | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 |
| Boric acid | 0.5 | 0.5 | 0.5 | 0.5 | 0.5 | 0.5 | 0.5 | 0.5 |
| Borax | 0.05 | 0.05 | 0.05 | 0.05 | 0.05 | 0.05 | 0.05 | 0.05 |
| Potassium sorbate | 0.1 | 0.1 | 0.1 | 0.1 | 0.1 | 0.1 | 0.1 | 0.1 |
| $\ell$-menthol | 0.005 | 0.005 | 0.005 | 0.005 | - | - | - | - |
| dl-camphor | - | - | - | - | 0.002 | 0.002 | 0.002 | 0.002 |
| NaCl | 0.275 | 0.275 | 0.275 | 0.085 | 0.275 | 0.275 | 0.275 | 0.085 |
| KCl | 0.055 | 0.055 | 0.055 | 0.017 | 0.055 | 0.055 | 0.055 | 0.017 |
| Propylene glycol | - | - | - | 0.5 | - | - | - | 0.5 |
| Effect for healing asthenopia | ◎◎ | ◎◎ | ◎◎ | ◎◎ | ◎◎ | ◎◎ | ◎◎ | ◎◎ |
| Test on compatibility with lenses | AC | AC | AC | AC | AC | AC | AC | AC |
| AC: Acceptable<br>* Incorporated proportion: w/w% | | | | | | | | |

Table 5

| Incorporated components | Sample No. | | | | | | | |
|---|---|---|---|---|---|---|---|---|
| | 29 | 30 | 31 | 32 | 33 | 34 | 35 | 36 |
| Marine Active | 1 | 1 | 1 | 1 | 1 | 1 | 1 | 1 |
| Px407 | 0.5 | - | - | - | 0.5 | - | - | - |
| Tetronic 1107 | - | 0.5 | - | - | - | 0.5 | - | - |
| Polysorbate | - | - | 0.25 | - | - | - | 0.25 | - |
| Hydroxypropyl-methylcellulose | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 |
| Boric acid | 0.5 | 0.5 | 0.5 | 0.5 | 0.5 | 0.5 | 0.5 | 0.5 |
| Borax | 0.05 | 0.05 | 0.05 | 0.05 | 0.05 | 0.05 | 0.05 | 0.05 |
| PHMB | 0.0001 | 0.0001 | 0.0001 | 0.0001 | 0.0001 | 0.0001 | 0.0001 | 0.0001 |
| $\ell$-menthol | 0.005 | 0.005 | 0.005 | 0.005 | 0.003 | 0.003 | 0.003 | 0.003 |
| dl-camphor | - | - | - | - | 0.002 | 0.002 | 0.002 | 0.002 |
| NaCl | 0.275 | 0.275 | 0.275 | 0.085 | 0.275 | 0.275 | 0.275 | 0.085 |
| KCl | 0.055 | 0.055 | 0.055 | 0.017 | 0.055 | 0.055 | 0.055 | 0.017 |
| Propylene glycol | - | - | - | 0.5 | - | - | - | 0.5 |
| Effect for healing asthenopia | ◎◎ | ◎◎ | ◎◎ | ◎◎ | ◎◎ | ◎◎ | ◎◎ | ◎◎ |

(continued)

| Incorporated components | Sample No. | | | | | | | |
|---|---|---|---|---|---|---|---|---|
| | 29 | 30 | 31 | 32 | 33 | 34 | 35 | 36 |
| Test on compatibility with lenses | AC | AC | AC | AC | AC | AC | AC | AC |
| AC: Acceptable<br>* Incorporated proportion: w/w% | | | | | | | | |

Table 6

| Incorporated components | Sample No. | | | | |
|---|---|---|---|---|---|
| | 37 | 38 | 39 | 40 | 41 |
| Hydroxypropyl-methylcellulose | - | 0.15 | - | - | - |
| Hydroxyethyl-cellulose | - | - | 0.15 | - | - |
| Sodium hyaluronate | - | - | - | 0.15 | - |
| Sodium chondroitin sulfate | - | - | - | - | 0.5 |
| EDTA·2Na | 0.05 | 0.05 | 0.05 | 0.05 | 0.05 |
| Boric acid | 0.5 | 0.5 | 0.5 | 0.5 | 0.5 |
| Borax | 0.05 | 0.05 | 0.05 | 0.05 | 0.05 |
| Potassium sorbate | 0.15 | 0.1 | 0.1 | 0.1 | 0.1 |
| NaCl | 0.275 | 0.275 | 0.275 | 0.275 | 0.275 |
| KCl | 0.055 | 0.055 | 0.055 | 0.055 | 0.055 |
| Effect for healing asthenopia | △ | △ | △ | △ | △ |
| Test on compatibility with lenses | AC | AC | AC | AC | AC |
| AC: Acceptable<br>* Incorporated proportion: w/w% | | | | | |

Table 7

| Incorporated components | Sample No. | | | | |
|---|---|---|---|---|---|
| | 42 | 43 | 44 | 45 | 46 |
| Histidine | 1 | - | - | - | - |
| Px407 | 0.5 | 0.5 | - | - | - |
| Tetronic 1107 | - | - | 0.5 | - | - |
| Polysorbate | - | - | - | 0.25 | - |
| Hydroxypropyl-methylcellulose | 0.15 | 0.15 | 0.15 | 0.15 | 0.15 |
| EDTA·2Na | 0.05 | 0.05 | 0.05 | 0.05 | 0.05 |
| Boric acid | 0.5 | 0.5 | 0.5 | 0.5 | 0.5 |
| Borax | 0.05 | 0.05 | 0.05 | 0.05 | 0.05 |
| Potassium sorbate | 0.1 | 0.1 | 0.1 | 0.1 | 0.1 |
| NaCl | 0.275 | 0.275 | 0.275 | 0.275 | 0.085 |
| KCl | 0.055 | 0.055 | 0.055 | 0.055 | 0.017 |

(continued)

| Incorporated components | Sample No. | | | | |
|---|---|---|---|---|---|
| | 42 | 43 | 44 | 45 | 46 |
| Propylene glycol | - | - | - | - | 0.5 |
| Effect for healing asthenopia | △ | △ | △ | △ | △ |
| Test on compatibility with lenses | AC | AC | AC | AC | AC |
| AC: Acceptable<br>* Incorporated proportion: w/w% | | | | | |

Table 8

| Incorporated components | Sample No. | | | | | | | |
|---|---|---|---|---|---|---|---|---|
| | 47 | 48 | 49 | 50 | 51 | 52 | 53 | 54 |
| Px407 | 0.5 | - | - | - | 0.5 | - | - | - |
| Tetronic 1107 | - | 0.5 | - | - | - | 0.5 | - | - |
| Polysorbate | - | - | 0.25 | - | - | - | 0.25 | - |
| Hydroxypropyl-methylcellulose | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 |
| Boric acid | 0.5 | 0.5 | 0.5 | 0.5 | 0.5 | 0.5 | 0.5 | 0.5 |
| Borax | 0.05 | 0.05 | 0.05 | 0.05 | 0.05 | 0.05 | 0.05 | 0.05 |
| Potassium sorbate | 0.1 | 0.1 | 0.1 | 0.1 | - | - | - | - |
| PHMB | - | - | - | - | 0.0001 | 0.0001 | 0.0001 | 0.0001 |
| $\ell$-menthol | 0.005 | 0.005 | 0.005 | 0.005 | 0.005 | 0.005 | 0.005 | 0.005 |
| NaCl | 0.275 | 0.275 | 0.275 | 0.085 | 0.275 | 0.275 | 0.275 | 0.085 |
| KCl | 0.055 | 0.055 | 0.055 | 0.017 | 0.055 | 0.055 | 0.055 | 0.017 |
| Propylene glycol | - | - | - | 0.5 | - | - | - | 0.5 |
| Effect for healing asthenopia | △ | △ | △ | △ | △ | △ | △ | △ |
| Test on compatibility with lenses | AC | AC | AC | AC | AC | AC | AC | AC |
| AC: Acceptable<br>* Incorporated proportion: w/w% | | | | | | | | |

As is evident from the results in Tables 1 to 8, liquid samples (Sample Nos. 1 to 36) having carnosine and/or anserine incorporated as a histidine derivative, were found to be capable of providing superior effects for healing asthenopia as compared with liquid samples (Sample Nos. 37 to 54) having no histidine derivative incorporated. Further, it was confirmed that among them, particularly with liquid samples (Sample Nos. 2 to 36) having a thickener and/or a surfactant added to the histidine derivative, the effect for healing asthenopia can further be improved than a liquid sample (Sample No. 1) wherein the histidine derivative was used alone.

Further, it was observed that even liquid samples (Sample Nos. 1 to 36) having a histidine derivative incorporated, present no influence over the shape (diameter) of a contact lens, like liquid samples (Sample Nos. 37 to 54) having no histidine derivative incorporated, whereby it can be understood that the ophthalmic composition of the present invention is advantageous in the compatibility with contact lenses. Accordingly, such an ophthalmic composition can be used irrespective of whether or not a contact lens is put on.

TEST EXAMPLE 2

As ophthalmic compositions intended particularly for cleaning, sterilizing, preserving or rinsing contact lenses, liquid samples (Sample Nos. 55 to 66) were prepared by incorporating the respective components in the proportions as identified in the following Table 9 to sterilized purified water and further having sodium hydroxide or hydrochloric acid suitably added to adjust the pH to 7.3. And, with respect to the obtained respective liquid samples, the test on the effect for healing asthenopia and the test on the compatibility with lenses were carried out in the same manner as in Test Example 1, and the obtained results are also shown in the following Table 9. Here, the test on the effect for healing asthenopia was carried out by a total of 20 volunteers comprising 10 rigid gas permeable hard contact lens users and 10 soft contact lens users, and the evaluation was carried out in the same manner as in Test Example 1.

Table 9

| Incorporated components | Sample No. | | | | | | | | | | | |
|---|---|---|---|---|---|---|---|---|---|---|---|---|
| | 55 | 56 | 57 | 58 | 59 | 60 | 61 | 62 | 63 | 64 | 65 | 66 |
| Marine Active | 2 | 2 | 2 | 2 | 2 | 2 | 2 | 2 | - | - | - | - |
| Px407 | - | - | 0.5 | - | 0.5 | - | - | - | 0.5 | - | 0.5 | - |
| Tetronic 1107 | - | - | - | 0.5 | - | 0.5 | - | - | - | 0.5 | - | 0.5 |
| Hydroxypropyl-methylcellulose | - | - | 0.15 | 0.15 | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 | 0.05 | 0.05 | 0.05 | 0.05 |
| Boric acid | - | - | 0.5 | 0.5 | - | - | 0.5 | - | 0.5 | 0.5 | - | - |
| Borax | - | - | 0.05 | 0.05 | - | - | 0.05 | - | 0.05 | 0.05 | - | - |
| Bis-Tris | 0.1 | 0.1 | - | - | 0.1 | 0.1 | - | 0.1 | - | - | 0.1 | 0.1 |
| NaCl | 0.1 | 0.1 | 0.1 | 0.1 | 0.1 | 0.1 | 0.1 | 0.1 | 0.45 | 0.45 | 0.45 | 0.45 |
| PHMB | 0.0005 | 0.0005 | 0.0005 | 0.0005 | 0.0005 | 0.0005 | 0.0005 | 0.0005 | 0.0005 | 0.0005 | 0.0005 | 0.0005 |
| Effect for healing asthenopia | ○ | ○ | ◎◎ | ◎◎ | ◎◎ | ◎◎ | ◎◎ | ◎◎ | △ | △ | △ | △ |
| Test on compatibility with lenses | AC | AC | AC | AC | AC | AC | AC | AC | AC | AC | AC | AC |

AC: Acceptable
* Incorporated proportion: w/w%

As is evident from the results in Table 9, liquid samples (Sample Nos. 55 to 62) having Marine Active i.e. a mixture of anserine and carnosine incorporated, were found to provide excellent effects for healing asthenopia. Particularly, it was confirmed that those (Sample Nos. 57 to 62) containing also a thickener and/or a surfactant, have better healing effects.

Further, it was confirmed that even liquid samples (Sample Nos. 55 to 62) having a histidine derivative incorporated as mentioned above, present no influence over the shape (diameter) of a contact lens. Accordingly, the ophthalmic composition of the present invention can be suitably used as an agent for contact lenses intended for cleaning, sterilizing, preserving, rinsing or the like of contact lenses.

TEST EXAMPLE 3

The following test was carried out to evaluate the effect for cleaning lipid and the sterilizing effect in a case where the ophthalmic composition of the present invention is used as an agent for contact lenses. Namely, firstly, in the same manner as in Test Examples 1 and 2, liquid samples (Sample Nos. 67 to 69) having the respective components incorporated in the proportions as identified in the following Table 10, to sterilized purified water and having the pH adjusted to 7.3, were prepared. Then, with respect to the obtained respective liquid samples, the following tests on the effect for cleaning lipid and on the sterilizing effect, were carried out.

Test on the effect for cleaning lipid

The effect for cleaning lipid, of the agent for contact lenses according to the present invention, was studied by a lipid solubilization rate method. Specifically, using a colored lipid prepared by mixing triglyceride (Pharmazol B-112, manufactured by NOF Corporation as lipid) and Sudan I (maximum absorption wavelength: 485.5 nm, manufactured by Nacalai Tesque Inc.) in a weight ratio of 99:1, 0.5 g thereof was put into a prescribed test bottle. Then, to such a test bottle, 20 ml of a liquid sample obtained as described above, was further added, and the opening of the test bottle was covered with a proper cover. Such an operation was carried out with respect to each liquid sample.

Then, each test bottle containing the colored lipid and the liquid sample, thus prepared, was shaked at a predetermined speed for 24 hours at a temperature of 25°C and then left to stand still for a predetermined period of time, whereupon the supernatant in each test bottle was sampled. With respect to each supernatant, the absorbance at a wavelength of 485.5 nm was measured by a spectrophotometer (self-recording spectrophotometer UV-2200, manufactured by Shimadzu Corporation). And, on the basis that the absorbance of the liquid sample (Sample No. 68) having no histidine derivative incorporated, is rated to be 1, the ratio of the absorbance (the absorbance ratio) of each of Sample Nos. 67 and 69, was calculated. The results are also shown in the following Table 10. Here, such an absorbance ratio being larger than 1, i.e. the absorbance being relatively high, means that the sample is superior in the effect for cleaning lipid specifically in the lipid solubilization performance.

Test on the sterilizing effect

The following test was carried out to examine the sterilization effect against bacteria, of the agent for contact lenses of the present invention. Namely, as one of test bacteria, Candida albicans (IFO 1594) was selected and was cultured at 32°C for 18 hours in a Sabourand glucose agar culture medium manufactured by Nippon Seiyaku K.K. Then, using the cultured bacteria, a suspension was prepared so that the number of bacteria would be from $10^7$ to $10^8$ cfu/ml, and 10 μℓ was added to 10 ml of each of separately prepared liquid samples (Sample Nos. 67 to 69). Then, each liquid sample having the suspension added, was maintained at room temperature for 6 hours, and then, the number of remaining live bacteria (cfu) was measured by a poured rate culture method. In this poured plate culture method, a Sabourand glucose agar culture medium was used as the culture medium, and culturing was carried out at 32°C for 3 days.

Further, as another test bacteria, Staphylococcus aureus (IFO 13276) was selected and cultured at 32°C for 18 hours in "SCD agar culture medium" tradename, manufactured by Nippon Seiyaku K.K. Then, using such cultured bacteria, a suspension was prepared so that the number of bacteria would be from $10^7$ to $10^8$ cfu/ml, and 100 μℓ was added to 10 ml of each of separately prepared liquid test samples. Then, each liquid sample having the suspension added, was maintained at room temperature for 20 minutes, and then the number of remaining live bacteria was measured by a poured plate culture method. Here, in the poured plate culture method, the above-mentioned SCD agar culture medium was used as the culture medium, and culturing was carried out at 32°C for 3 days.

Then, with respect to each test bacteria, the number of remaining bacteria was measured, and the logarithmic decrement of such test bacteria, was calculated in accordance with the following formula. The logarithmic decrement calculated by this formula is also shown in the following Table 10.

Logarithmic decrement=log (number of inoculated

bacteria)-log(number of remaining bacteria)

Table 10

| Incorporated components | | Sample No. | | |
|---|---|---|---|---|
| | | 67 | 68 | 69 |
| Marine Active | | 1 | - | 1 |
| Px407 | | 0.5 | 0.5 | - |
| Hydroxypropyl-methylcellulose | | 0.15 | 0.15 | 0.15 |
| EDTA·2Na | | 0.05 | 0.05 | 0.05 |
| Boric acid | | 0.5 | 0.5 | 0.5 |
| Borax | | 0.05 | 0.05 | 0.05 |
| NaCl | | 0.275 | 0.45 | 0.275 |
| KCl | | 0.055 | 0.09 | 0.055 |
| PHMB | | 0.0005 | 0.0005 | 0.0005 |
| Absorbance ratio | | 1.05 | 1 | 0.03 |
| Logarithmic decrement | C. albicans (Upon expiration of 4 hrs) | >3.16 | >3.16 | >3.16 |
| | S. aureus (Upon expiration of 4 hrs) | >3.96 | >3.96 | >3.96 |

* Incorporated proportion: w/w%

As is evident from the results in Table 10, Sample No. 67 having a histidine derivative incorporated is capable of exhibiting the same levels of lipid solubilization performance and sterilizing effect as the liquid sample (Sample No. 68) having no histidine derivative incorporated. It was thereby confirmed that in the agent for contact lenses according to the present invention, the histidine derivative effective for healing asthenopia will present no adverse effects to the cleaning effects or the sterilizing effects by other added components.

As is evident from the foregoing description, the ophthalmic composition of the present invention has at least one histidine derivative which is safe to living organisms and provides excellent effects for healing asthenopia, incorporated in an aqueous medium, whereby it is free from presenting adverse effects to living organisms even when used for a long period of time and is capable of providing excellent effects for healing asthenopia.

Further, such an ophthalmic composition will not change the shape of a contact lens, and it can be applied as e.g. eye drop to an eye suffering from asthenopia either when a contact lens is put on or when no contact lens is put on. Particularly in a case where it is applied when a contact lens is put on, in addition to the above-described healing asthenopia, the comfortableness in wearing the contact lens can also advantageously be improved.

Further, a case where the ophthalmic composition of the present invention is used as an agent for contact lenses, it will not present any adverse effect to the effect for cleaning lipid and the sterilizing effect of the agent for contact lenses, whereby in addition to the effect for cleaning lipid and the sterilizing effect, the effect for healing asthenopia can be obtained.

## Claims

1. An ophthalmic composition useful as an agent for contact lenses or as eye drops to be applied while a contact lens is put on,
   wherein at least one histidine derivative is incorporated in an aqueous medium, **characterized in that** the histidine derivative is anserine incorporated in a proportion of from 0.001 to 5 w/w%.

2. The ophthalmic composition according to Claim 1, wherein as a further histidine derivative carnosine is incorporated in a proportion of from 0.0001 to 3 w/w%.

3. The ophthalmic composition according to any one of Claims 1 or 2, which further contains a thickener and/or a surfactant.

4. The ophthalmic composition according to any one of Claims 1 to 3, which further contains at least one member selected from the group consisting of antiseptics, buffer agents, chelating agents, isotonic agents, refrigerants, antiinflammatory, vitamins and amino acids.

5. An eye drop **characterized in that** it is composed of the ophthalmic composition as defined in any one of Claims 1 to 4.

6. An agent for contact lenses, **characterized in that** it is composed of the ophthalmic composition as defined in any one of Claims 1 to 4.

7. Use of an ophthalmic composition wherein at least one histidine derivative is incorporated in an aqueous medium, for the preparation of a medicament for the treatment of asthenopia,
   wherein said histidine derivative is anserine or carnosine.

8. The use according to claim 7, wherein the composition is as defined in any one of claims 1 to 4.

9. Use of an ophthalmic composition as defined in any one of claims 1 to 4 for the preparation of an agent for contact lenses.

## Patentansprüche

1. Ophthalmische Zusammensetzung, die als Mittel für Kontaktlinsen oder als Augentropfen, die während des Einsetzens einer Kontaktlinse appliziert werden, brauchbar ist,
   worin mindestens ein Histidin-Derivat in einem wässerigen Medium enthalten ist, **dadurch gekennzeichnet, dass** das Histidin-Derivat in einem Anteil von 0,001 bis 5 Gew./Gew.-% enthaltenes Anserin ist.

2. Ophthalmische Zusammensetzung nach Anspruch 1, worin als weiteres Histidin-Derivat Carnosin in einem Anteil von 0,0001 bis 3 Gew./Gew.-% enthalten ist.

3. Ophthalmische Zusammensetzung nach einem der Ansprüche 1 oder 2, die ferner ein Verdickungsmittel und/oder ein oberflächenaktives Mittel enthält.

4. Ophthalmische Zusammensetzung nach einem der Ansprüche 1 bis 3, die ferner mindestens eine Substanz enthält, ausgewählt aus der Gruppe bestehend aus Antiseptika, Puffermitteln, Chelatbildnern, isotonischen Mitteln, Kühlmitteln, entzündungshemmenden Mitteln, Vitaminen und Aminosäuren.

5. Augentropfen, **dadurch gekennzeichnet, dass** sie aus der in einem der Ansprüche 1 bis 4 definierten ophthalmischen Zusammensetzung bestehen.

**6.** Mittel für Kontaktlinsen, **dadurch gekennzeichnet, dass** es aus der in den Ansprüchen 1 bis 4 definierten ophthalmischen Zusammensetzung besteht.

**7.** Verwendung einer ophthalmischen Zusammensetzung, worin in einem wässerigen Medium mindestens ein Histidin-Derivat eingebaut ist, zur Herstellung eines Arzneimittels zur Behandlung von Asthenopie, worin das Histidin-Derivat Anserin oder Carnosin ist.

**8.** Verwendung nach Anspruch 7, worin die Zusammensetzung eine wie in einem der Ansprüche 1 bis 4 definierte Zusammensetzung ist.

**9.** Verwendung einer ophthalmischen Zusammensetzung, wie in einem der Ansprüche 1 bis 4 definiert, zur Herstellung eines Mittels für Kontaktlinsen.

**Revendications**

**1.** Composition ophtalmique utile en tant qu'agent pour lentilles de contact ou en tant que collyres à appliquer alors que l'on met des lentilles de contact, dans laquelle au moins un dérivé d'histidine est incorporé dans un milieu aqueux, **caractérisée en ce que** le dérivé d'histidine est l'ansérine, incorporée en une proportion de 0,001 à 5% p/p.

**2.** Composition ophtalmique selon la revendication 1, dans laquelle en tant que dérivé d'histidine additionnel est incorporée de la carnosine en une proportion de 0,0001 à 3% p/p.

**3.** Composition ophtalmique selon l'une quelconque des revendications 1 et 2, qui contient en outre un épaississant et/ou un surfactant.

**4.** Composition ophtalmique selon l'une quelconque des revendications 1 à 3, qui contient encore au moins un membre choisi dans le groupe constitué par des antiseptiques, des agents tampons, des agents chélatants, des agents isotoniques, des réfrigérants, des anti-inflammatoires, des vitamines et des acides aminés.

**5.** Collyre **caractérisé en ce qu'**il est composé de la composition ophtalmique définie dans l'une quelconque des revendications 1 à 4.

**6.** Agent pour lentilles de contact, **caractérisé en ce qu'**il est composé de la composition ophtalmique définie dans l'une quelconque des revendications 1 à 4.

**7.** Utilisation d'une composition ophtalmique dans laquelle au moins un dérivé d'histidine est incorporé dans un milieu aqueux, pour la préparation d'un médicament pour le traitement de l'asthénopie, ledit dérivé d'histidine étant l'ansérine ou la carnosine.

**8.** Utilisation selon la revendication 7, dans laquelle la composition est telle que définie dans l'une quelconque des revendications 1 à 4.

**9.** Utilisation d'une composition ophtalmique telle que définie dans l'une quelconque des revendications 1 à 4, pour la préparation d'un agent pour lentilles de contact.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9040549 A
- JP 9143064 A
- JP 2000247885 A

**Non-patent literature cited in the description**

- *Biochemistry (Moscow,* 2000, vol. 65, 588-598
- *Clinica Chimica Acta,* 1996, vol. 254, 1-21
- *Vestnik oftalmologii.,* 1997, vol. 113, 27-31
- Marine Active. **L-CARNOSINE.** tradename, manufactured. Kagaku Industry Co., Ltd,